(19) Europäisches Patentamt
European Patent Office
Office européen des brevets

(11) **EP 2 655 652 B1**

(12) **FASCICULE DE BREVET EUROPEEN**

(45) Date de publication et mention
de la délivrance du brevet:
**03.06.2015 Bulletin 2015/23**

(51) Int Cl.:
*C12Q 1/02* (2006.01)  *G01N 33/483* (2006.01)

(21) Numéro de dépôt: **11815510.0**

(22) Date de dépôt: **21.12.2011**

(86) Numéro de dépôt international:
**PCT/FR2011/053138**

(87) Numéro de publication internationale:
**WO 2012/085468 (28.06.2012 Gazette 2012/26)**

(54) **METHODE POUR MESURER LA RESISTANCE DE FILMS**

VERFAHREN ZUR MESSUNG DES WIDERSTANDES VON FOLIEN

METHOD FOR MEASURING THE RESISTANCE OF FILMS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **21.12.2010 FR 1060960**

(43) Date de publication de la demande:
**30.10.2013 Bulletin 2013/44**

(73) Titulaire: **Biofilm Control
63360 Saint Beauzire (FR)**

(72) Inventeurs:
• **BERNARDI, Thierry
F-63170 Perignat Les Sarlieve (FR)**
• **MAYER, Pascal
F-63200 Marsat (FR)**
• **GROELLY, Jérôme
F-63110 Beaumont (FR)**

(74) Mandataire: **Novagraaf Technologies
Bâtiment O2
2, rue Sarah Bernhardt
CS90017
92665 Asnières-sur-Seine Cedex (FR)**

(56) Documents cités:
**FR-A1- 2 916 761**

• **CHAVANT ET AL: "A new device for rapid
evaluation of biofilm formation potential by
bacteria", JOURNAL OF MICROBIOLOGICAL
METHODS, vol. 68, no. 3, 16 février 2007
(2007-02-16), pages 605-612, XP005892101, ISSN:
0167-7012, DOI: 10.1016/J.MIMET.2006.11.010**
• **LARSON F ET AL: "Surface adhesion
measurements in aquatic biofilms using
magnetic particle induction: MagPI",
LIMNOLOGY AND OCEANOGRAPHY:
METHODS, vol. 7, juillet 2009 (2009-07), pages
490-497, XP055004171, ISSN: 1541-5856, DOI:
10.4319/lom.2009.7.490**

## Description

### Domaine technique

La présente invention se rapporte à un procédé de mesure de l'impact d'une action sur un film. En particulier, la présente invention se rapport à un procédé de mesure de l'effet d'une action, par exemple mécanique, hydrodynamique, physique, chimique ou biologique sur l'intégrité d'un film.

La présente invention trouve notamment une application dans le domaine de la biologie, chimie, biotechnologie.

Dans la description ci-dessous, les références entre crochets (**[Ref.]**) renvoient à la liste des références présentée à la fin du texte.

### Etat de la technique

Il existe une multitude de dépôts ou de couches fines de substances inertes ou vivantes (biologiques) liées à des supports : par exemple des peintures, des vernis, des films composés de macromolécules, des biofilms de microorganismes et de cellules diverses, par exemple des bactéries, levures, algues, cellules d'organismes multicellulaires. Dans la suite, nous désignerons ces couches et dépôt sur un support par le terme de " film ".

Parmi ces films, un grand nombre se forme de façon progressive au cours du temps, par exemple par polymérisation de monomères, par agrégation ou d'interpénétration de macromolécules, par développement de biofilms de microorganismes, par multiplication cellulaire et/ou production de substances liant les cellules entre-elles.

Cette formation peut souvent être modulée par diverses substances ou paramètres physiques dont il est utile de connaître l'impact. Un film peut aussi être modifié après sa formation par l'action d'un traitement physico-chimique, par exemple par des détergents, solvants, rayonnements, par exemple, action thermique, etc...

Toutes ces actions peuvent conduire à des films dont on peut être intéressé à connaître la résistance à une action hydrodynamique, tel un jet, un flux ou l'agitation d'une solution qui recouvre le film. Par exemple, on peut aussi simplement s'intéresser à savoir si ce film a pu encore se former ou s'il s'est démantelé. On désignera dans la suite de ce document ces propriétés par le terme « intégrité ».

Des procédés connus permettent de comparer l'intégrité de films de différentes natures, obtenus par différents modes de formation et résultant de différents traitements.

Par exemple dans un des procédés connus un support dont la couleur est différente de celle du film est utilisé. Ainsi, lorsque les films sont formés sur ce support, il est aisé pour l'homme de l'art d'en mesurer l'intégrité après, par exemple une action hydrodynamique, un traitement physico-chimique, et/ou une modulation de sa formation par l'analyse d'une prise d'image.

Toutefois, il existe également des films transparents ou ne pouvant être coloré, par exemple les films vivants, les films formés par des microorganismes, les films issus de polymérisation ou d'agrégation pour lesquels la substance utilisée pour la coloration peut interférer avec leur formation.

Il existe des procédés dans l'état de la technique afin de mesurer l'intégrité d'un film à partir, par exemple de ses propriétés physiques, tels que son indice de réfraction, par exemple dans Singh et al, Physica Scripta. Vol. 65, 167-180 (2002) : « Refractive Index Measurement and its Applications » [Ref. 1] et dans Vôrôs et al. Biophysical Journal, Vol. 87 , 553-561 (2004) : « The Density and Refractive Index of Adsorbing Protein Layers» [Ref. 2].

Cependant, ces procédés sont des procédés couteux qui nécessitent une formation et un personnel qualifié ainsi que des instruments sophistiqués et couteux.

Il y a des situations, par exemple dans le domaine médical ou le domaine biologique, où il est nécessaire mesurer l'intégrité d'un grand nombre de films différents en présence de composés chimiques, de molécules biologiques, de modifications thermodynamiques etc. Il peut s'agir par exemple d'évaluer l'efficacité d'un composé sur des biofilms ou encore l'action, par exemple la dégradation, la modification ou l'efficacité d'une enzyme sur un film de polymères.

Dans ces situations, par exemple à l'occasion de campagnes de criblage de molécules ou d'enzymes actives contre des biofilms, par exemple pour l'estimation de l'efficacité d'antibiotiques sur des biofilms dans le cadre de soins médicaux, les techniques décrites précédemment ne sont pas satisfaisante car elles ne permettent pas à l'homme de l'art de réaliser les mesures à un rythme suffisamment rapide.

En outre, les procédés connus nécessitent l'utilisation d'un grand nombre d'instruments qui ne peuvent être utilisés simultanément dans un procédé de criblage. De plus, les instruments utilisés sont couteux, et nécessite la présence de personnel qualifié.

En outre, le temps pour obtenir les résultats avec les procédés connus est très important et peut nécessiter l'adjonction de réactifs susceptible de modifier le résultat et donc d'entrainer une variabilité des résultats et la non reproductions de ceux-ci.

Il existe donc un réel besoin de trouver un procéder de mesure de l'effet d'une action sur un film palliant ces défauts, inconvénients et obstacles de l'art antérieur, en particulier d'un procédé permettant de maîtriser le temps, de réduire l'instrumentation utile pour ce procédé, de réduire les coûts et d'améliorer la détection de l'effet.

Le document JOURNAL OF MICROBIOLOGICAL METHODS, vol. 68, no. 3, pages 605-612, 2007, XP005892101, ISSN 0167-7012 décrit un dispositif et un procédé pour une évaluation rapide de la formation d'un biofilm potentiel par une bactérie comprenant l'utilisation des particules magnétiques et l'application d'une force magnétique.

Le document FR 2 916 761 A1 décrit un procédé de détermination de la sensibilité d'un microorganisme à un antibiotique susceptible d'empêcher le développement de biofilm comprenant lui aussi l'utilisation des particules magnétiques et l'application d'une force magnétique.

Aucun de ces documents ne divulgue le regroupement des particules sur une surface immergée avant la formation du biofilm.

**Description de l'invention**

Le procédé de la présente invention permet de résoudre les défauts, inconvénients et obstacles de l'art antérieur précités.

En particulier, la présente invention a pour objet un procédé de mesure de l'effet d'au moins une action sur un film comprenant les étapes suivantes.

    a. introduction dans une solution d'au moins une substance capable de former un film,
    b. introduction dans la solution obtenue en (a) d'au moins deux particules, lesdites particules reposant sur une surface immergée S dans ladite solution,
    c. regroupement des particules sur ladite surface immergée S, lesdites particules formant sur ladite surface un point ou une tache,
    d. formation dudit film à partir de ladite substance,
    e. observation du point ou de la tache sur la surface S,
    f. application d'une action mécanique et/ou physique à ladite solution,
    g. observation de l'effet sur le film de l'action appliquée à l'étape (f) par observation du point ou de la tache sur la surface S, et
    h. détermination de l'effet de l'action appliquée sur le film par comparaison des observations des étapes (e) et (g) précitées.

Selon l'invention une substance capable de former un film peut être, par exemple des microorganismes, des aliments, des substances chimiques, des macromolécules synthétiques, des macromolécules biologiques, des colloïdes et des émulsions, des objets de taille microscopique et des objets de taille nanoscopique.

Il peut s'agir par exemple de cellules eucaryotes, par exemple des cellules eucaryotes animales, par exemples des cellules du sang, par exemple des leucocytes, par exemple des granulocytes, des polynucléaires neutrophiles ; des polynucléaires éosinophiles ; des polynucléaires basophiles ; des lymphocytes B, des lymphocytes T, des lymphocytes NK, des monocytes, des érythrocytes, des thrombocytes. Il peut s'agir également de cellules eucaryotes végétales, par exemple des cellules de l'épiderme végétal, du xylème, du phloème, du parenchyme, du collenchyme, du sclérenchyme. Il peut s'agir également de champignons, de levures. Il peut s'agir par exemple *Candida, Cryptococcus, Malassezia, Pityrosporum, Pneumocystis, Epidermo-phyton, Microsporum, Trichophyton.* Il peut s'agir également de protozoaires, par exemple *Entamoeba histolytica, Acanthamoeba castellanii, Naegleria fowleri.*

Il peut s'agir également de cellules procaryotes, par exemple toute bactérie connu de l'homme du métier, par exemple les bactéries comprises dans le groupe, sans être limité à celui-ci, constitué de Acetobacter aurantius, Actinobacillus actinomycetemcomitans, Agrobacterium tumefaciens, Azorhizobium caulinodans, Azotobacter vinelandii, Bacillus anthracis, Bacillus brevis, Bacillus cereus, Bacillus fusiformis, Bacillus licheniformis, Bacillus megaterium, Bacillus stearothermophilus, Bacillus subtilis, Bacteroides gingivalis, Bacteroides melaninogenicus, Bartonella henselae, Bartonella quintana, Bordetella bronchiseptica, Bordetella pertussis, Borrelia burgdorferi, Branhamella catarrhalis, Brucella abortus, Brucella melitensis, Brucella suis, Burkholderia mallei, Burkholderia pseudomallei Calymmatobacterium granulomatis, Campylobacter coli, Campylobacter jejuni, Campylobacter pylori, Chlamydia pneumoniae, Chlamydia psittaci, Chlamydia trachomatis, Chlamydophila pneumoniae, Chlamydophila psittaci, Clostridium botulinum, Clostridium difficile, Clostridium perfringens, Clostridium tetani, Clostridium welchii, Corynebacterium diphtheriae, Corynebacterium fusiforme, Coxiella burnetii Ehrlichia chaffeensis, Enterococcus avium, Enterococcus durans, Enterococcus faecalis, Enterococcus faecium, Enterococcus galilinarum, Enterococcus maloratus, Escherichia coli Francisella tularensis, Fusobacterium nucleatum Gardnerella vaginalis Haemophilus ducreyi, Haemophilus influenzae, Haemophilus parainfluenzae, Haemophilus pertussis, Haemophilus vaginalis, Helicobacter pylori Klebsiella pneumoniae, Klebseilla rhinoscleromatis-klebsiella oxytoca Lactobacillus acidophilus, Lactobacillus casei, Lactococcus lactis, Legionella pneumophila, Methanobacterium extroquens, Microbacterium multiforme, Micrococcus luteus, Mycobacterium avium, Mycobacterium bovis, Mycobacterium diphtheriae, Mycobacterium intracellulare, Mycobacterium leprae, Mycobacterium lepraemurium, Mycobacterium phlei, Mycobacterium smegmatis, Mycobacterium tuberculosis, Mycoplasma fermentans, Mycoplasma genitalium, Mycoplasma hominis, Mycoplasma pneumoniae Neisseria gonorrhoeae, Neisseria meningitidis, Nocardia asteroides Pasteurella multocida, Pasteurella tularensis, Porphyromonas gingivalis, Pseudomonas aeruginosa, Pseudomonas maltophilia, Rhizobium radiobacter, Rickettsia prowazekii, Rickettsia mooseri, Rickettsia psittaci, Rickettsia quintana, Rickettsia rickettsii, Rickettsia trachomae, Rochalimaea henselae, Rochalimaea quintana, Rothia dentocariosa, Salmonella enteritidis, Salmonella typhi, Salmonella typhimurium, Serratia marcescens, Shigella dysenteriae, Staphylococcus aureus, Staphylococcus epidermidis, Streptococcus agalactiae, Streptococcus avium, Streptococcus bovis, Streptococcus cricetus, Streptococcus faceium, Streptococcus faecalis, Streptococcus ferus, Streptococcus gallinarum, Streptococcus lactis, Streptococcus mitior, Streptococcus mitis, Streptococcus mutans, Streptococcus oralis, Streptococcus pneumoniae, Streptococcus pyogenes, Streptococcus rattus, Streptococcus salivarius, Streptococcus sanguis, Streptococcus sobrinus, Treponema pallidum, Vibrio cholerae, Vibrio comma, Vibrio parahemolyticus, Vibrio vulnificus, Xanthomonas maltophilia Yersinia enterocolitica,Yersinia pestis et Yersinia pseudotuberculosis, etc.

Par colloïdes et émulsions on entend une substance sous forme de liquide ou semi-solide qui contient des particules suffisamment petites pour que le mélange soit homogène. Il peut s'agir de tout colloïde et émulsion connu de l'homme du métier. Il peut s'agir par exemple de toute substance et/ ou composition comportant deux phases distinctes. Il peut s'agir, par exemple, de liquide contenant en suspension des particules, par exemple des liposomes, des gouttelettes, des agrégats, qui peuvent avoir une taille particulaire, par exemple de 2 à 200 nanomètres, par exemple de 201 nm à 5$\mu$m. Il peut s'agir par exemple de nanoémulsions, de lait, de crème de lait, de beurre, de mayonnaise, de crème hydratante, d'argiles, d'or ou d'argent colloïdal, d'émulsion synthétiques d'huile dans l'eau, de ferrro-fluide dans l'eau, d'eau dans l'huile.

Selon l'invention par macromolécules synthétiques on entend des molécules chimiques et/ou naturelles modifiées avec un poids moléculaire élevé, par exemple un poids de 1 à 1000kDa, par exemple du polystyrène sulfonate, du polyéthylène glycol,

Selon l'invention par macromolécules biologiques on entend toutes biologiques avec un poids moléculaire élevé, par exemple un poids de 5 à 1000kDa. Il peut s'agir par exemple d'assemblage, par exemple par liaison covalente, de molécules naturelles, des acides nucléiques tels que l'ADN et l'ARN, des polysacharadies tels que le dextrane, la celluloise, l'amidon, des protéines tels que l'actine, le fibrinogène et la fibrine.

Selon l'invention par objets de taille microscopique on entend toute substance et/ou objet connu de l'homme du métier dont la taille est de 1 à 1000$\mu$m, de préférence de 1 à 100$\mu$m.

Selon l'invention par objets de taille nanoscopique. on entend toute particule connue de l'homme du métier dont la taille est inférieure à 1 $\mu$m et/ou 1000 nm, par exemple de 50nm à 950nm, de 1 à 100 nm.

Selon l'invention, le film peut-être formé, par exemple par polymérisation de monomères via des liaisons covalentes, par multimérisation de protéines via des liaisons covalentes, par exemple des ponts disulfure, des liaisons peptiques, via des liaisons non-covalentes, par exemple des ponts salins, des liaisons hydrogène, des forces de Van der Waals, par la sédimentation des micro-organismes, par multiplication de micro-organismes sur une surface, par la sécrétion de polymères par ces micro-organismes, par exemple des acides nucléiques, des protéines, des polysaccharides, par polymérisation de protéines, par exemple de protéines plasmatiques, de protéines cellulaires.

Le film peut-être également formé par la polymérisation de monomères, par exemple d'acrylamide, de bisacrylamide, d'éthylène, de propylène, de vinyle, d'amino-acides ;par la déposition d'une émulsion sur une surface, par exemple une peinture, un verni par la formation d'un d'hydro-gels ou d'aérogels, par exemple un gel d'agarose, d'agar, de gélatine, par exemple un aérogel de silice, d'alumine, d'oxyde de chrome (III) ou d'oxyde d'étain, de SeaGel TM.

Selon l'invention, le film peut-être également formé par évaporation / lyophilisation de solutions, par exemple une solution de protéines, d'ADN, de graisse diluée dans un solvant, par exemple d'émulsions, par exemple une peinture, un verni, par changement de phase liquides-solide induit, par exemple, par un changement de température, par exemple avec du beurre fondu, une crème hydratante, une graisse.

Selon l'invention, l'action appliquée peut être, par exemple une action mécanique, hydrodynamique ou physique.

Selon l'invention, l'application peut être réalisée, par exemple de 1 à 24 heures, de 1 à 60 minutes, de 1 à 5 minutes, de 1 à 60 secondes.

Selon l'invention, par actions mécaniques on entend par exemple, l'application d'une brosse, d'une spatule, d'un disque soumis à un mouvement alternatif le long d'une surface, à un mouvement rotatif ou circulaire, à une pression.

Selon l'invention, par actions hydrodynamiques on entend par exemple, la mise en mouvement en rotation, par exemple à l'aide d'un agitateur, par exemple à une vitesse de rotation de 0,1 à 1000 tpm, de 5 à 300 tpm, et/ou l'application d'un jet de liquide ou de gaz, par exemple avec une pompe, un jet de liquide ou de gaz peut être par exemple à une pression de 1,01 à 10 bars, de préférence de 1,1 à 2.bars.

Selon l'invention, par actions physiques on entend par exemple une irradiation, par exemple par un rayonnement électromagnétique, par exemple l'application d'un faisceau lumineux de longueur d'onde de 10nm à 10$\mu$m, de 100 nm à 1 $\mu$m. Le faisceau peut avoir une intensité, par exemple de 0,01W à 100W, de 0,1W à 10 W. Il peut s'agir également, par exemple d'un bombardement de particules, par exemple avec des particules nucléaires, par exemple des neutrons, électrons, particules accélérées issues d'un accélérateur de particule, d'une source radioactive. par exemple de grains de matière, par exemple de sable, par exemple d'un diamètre de 50 à 500 $\mu$m. de sel, par exemple d'un diamètre de 50.à 500$\mu$m, de métal, par exemple de cuivre, de fer, de zinc, d'aluminium, par exemple d'un diamètre de 10 à 500$\mu$m.

De préférence, ladite au moins une action appliquée dans le procédé de l'invention est une action hydrodynamique.

Selon l'invention, le procédé peut comprendre, indépendamment, l'application d'au moins deux, au moins trois actions décrites précédemment. Par exemple, le procédé de l'invention peut comprendre l'application d'une action hydrodynamique et d'une action électromagnétique, par exemple d'une irradiation aux rayons ultra-violets suivi, par exemple d'une mise en mouvement par rotation, d'une irradiation avec des particules béta suivi, par exemple de l'application d'un jet d'eau.

La solution susceptible d'être utilisée dans la présente invention peut être, par exemple une solution liquide ou un gaz. La solution peut être toute solution connue de l'homme du métier. Il peut s'agir par exemple d'un milieu de culture, par exemple un milieu de culture de cellules eucaryotes et/ou procaryotes, un milieu tampon, par exemple tout milieu tampon connu de l'homme du métier, par exemple un milieu tampon disponible dans le commerce, par exemple du tampon phosphate salin (PBS), un échantillon biologique, par exemple un échantillon de sang, de plasma, d'urine, de liquide céphalorachidien, une solution saline, par exemple une solution physiologique, un milieu de culture, par exemple de l'infusion de coeurs et de cerveaux disponible dans le commerce, d'un solvant, par exemple de l'acétone, du diméthylsulfoxyde, de l'éthanol, du méthanol, du propanol, de l'acétonitrile, l'acétate d'éthyle, l'éther, du phénol, du chloroforme, du tétrahydrofurane, du difluoroéthylène, et/ou un hydrocarbure, par exemple de l'hexane, du cyclo-hexane, du benzène, de l'octane, du décane, du pétrole, de l'essence, du gasoil.

Selon l'invention, le gaz peut être par exemple de l'air, de l'oxygène, de l'azote, du néon, de l'argon, du gaz-carbonique, du méthane, de l'ozone.

Selon l'invention une solution liquide peut avoir une masse volumique de 0,1 à 4 kg/l, de 0,3 à 3 kg/l; une solution gazeuse peut avoir une densité de $10^{-15}$ kg/m$^3$ à 1000 kg/m$^3$, de $10^{-10}$ à 30 kg/m$^3$, de $10^{-5}$ à 3 kg /m$^3$.

L'homme du métier de part ces connaissances générales saura aisément déterminer la masse volumique d'une solution. Par exemple la mesure de la masse volumique de la solution peut être réalisée par exemple par mesure du ratio de la masse sur le volume, par exemple en pesant une solution de volume connue.

Selon l'invention, la solution peut être préalablement traitée, par exemple, la solution peut être purifiée, diluée, concentrée.

Selon l'invention, la solution peut être purifiée par tout procédé connu de l'homme du métier, par exemple par dialyse, par filtration, par ultrafiltration, par clarification et par centrifugation. Par exemple, le procédé de filtration peut comprendre le passage de la solution sur un tamis avec des pores de 0,2 à 100 $\mu$m, le procédé d'ultrafiltration peut comprendre, par exemple une centrifugation à une vitesse de 1 à 3000 tours par minute pendant un temps de 0,1 à 30 minutes, le procédé de dialyse peut être, par exemple un procédé comprenant une étape de dépôt de la solution sur une membrane de dialyse, par exemple à seuil de coupure de 500 Da, ladite membrane flottant sur de l'eau distillée contenue dans un récipient. Le procédé de clarification peut être, par exemple un procédé comprenant l'ajout dans la solution de 0,1% (poids/poids) d'albumine de sérum de bovin.

Selon l'invention, la purification de la solution peut permettre, avantageusement, d'éliminer de la solution tout contaminant et/ou molécules susceptibles d'altérer la détermination de l'effet, par exemple la purification peut permettre d'éliminer indépendamment des bactéries, des virus, des protéines, des molécules chimiques, des sels, des grains de matières, des agrégats de molécules. Bien entendu, l'homme du métier de part ces connaissances générales saura adapter le procédé de purification en fonction de la solution

Selon l'invention, la solution peut être également diluée, par exemple par tout procédé connu de l'homme du métier, par exemple par dilution sérielle. La dilution peut être réalisée avec tout diluant connu de l'homme du métier. Il peut s'agir par exemple d'une solution tampon, par exemple du tampon phosphate salin, une solution saline, par exemple du sérum physiologique, de l'éthanol, du DMSO, de l'acétone, de l'hexane et/ou tout solvant, hydrocarbure ou solution décrite précédemment.

La solution peut être diluée, par exemple d'un facteur de 2 à 20000, de 5 à 500, de 5 à 50.

La dilution de la solution peut permettre, avantageusement, de modifier la concentration des composants présents dans la solution, par exemple, en diminuer la concentration, par exemple la dilution peut permettre de diminuer la concentration protéique. La dilution peut permettre ainsi de diminuer la concentration d'éventuels composés interférents et ainsi avantageusement d'améliorer la spécificité et/ou la sensibilité du procédé de l'invention.

Selon l'invention, la solution peut être également concentrée, par exemple par tout procédé connu de l'homme du métier, par exemple par ultracentrifugation, par ultrafiltration, par évaporation, par lyophilisation

Selon l'invention, la purification, dilution et/ou concentration de ladite solution peut avantageusement permettre d'ajuster la masse volumique de ladite solution.

L'ajustement de la masse volumique de la solution permet avantageusement d'améliorer la sensibilité du procédé de l'invention, notamment en augmentant, en diminuant ou en annulant l'effet de la force de pesanteur qui pousse les particules vers la surface.

Selon l'invention, le volume de la solution utilisée dans le procédé peut être, par exemple de 0,3 $\mu$l à 100 ml, de 3$\mu$l à 10ml, de 30 $\mu$l à 1 ml.

Selon l'invention, l'incubation de la solution peut être réalisée, par exemple à une température comprise de -10°C à 90°C, de 0°C à 40°C, de 15°C à 25°C.

Selon l'invention, le temps d'incubation peut être réalisé, par exemple de 1 à 72 heures, de 2 à 48 heures, de 1 à 24 heures, de 1 à 60 minutes.

Selon l'invention par effet on entend, par exemple l'abrasion, la déchirure, par exemple une déchirure totale ou partielle, la déstructuration, la destruction, l'arrachement, le détachement, le perçage, l'apparition de fissures, de crevasses, de trous, de pores, le gonflement, la contraction et/ou l'inhibition de la formation du film.

Selon l'invention, le procédé de l'invention peut être mis en oeuvre avec une pluralité de particules, par exemple avec au moins 2 particules, avec par exemple de 2 à 10 000 000, de 1000 à 1 000 000, de de 10 000 à 1 000 000, de 100 000 à 1 000 000, de 10 000 à 100 000. La pluralité de particules permet avantageusement de détecter directement, sans dispositif complexe de visualisation et sans colorant, l'interaction entre lesdites substances au contraire des procédés de l'art antérieur utilisant une seule particule et nécessitant pour la détection de l'interaction des dispositifs complexes de visualisation ou des colorants.

Selon l'invention, lesdites, au moins deux, particules, peuvent être choisies dans le groupe comprenant des particules chargées électriquement, des particules magnétiques, des particules revêtues d'au moins une couche magnétique, des particules magnétisables, des particules revêtues d'une couche magnétisable, des particules électriques, électromagnétiques, électrisables, portant une charge électrique ou un mélange de deux ou plusieurs de ces particules. En fait, il peut s'agir de toute particule permettant de mettre en oeuvre la présente invention.

Avantageusement, lesdites particules peuvent être une particule de toute forme adaptée à la mise en oeuvre de la présente invention, par exemple sous forme de bille, de palet, de forme géométrique asymétrique, par exemple avec une face plane, etc.

Toute taille appropriée de particule magnétique peut-être utilisée. La taille peut être choisie par exemple en fonction de la taille du contenant de la solution. Par exemple, la taille des particules peut être inférieure au dixième de la taille du contenant, de préférence inférieure au centième, de manière encore plus préférée inférieur au millième de la taille du contenant. Par exemple la particule peut présenter une taille de, par exemple, 10 nm à 100 $\mu$m, de 0,1 à 10 $\mu$m.

Selon l'invention, les particules peuvent être éclairées, par exemple au moyen d'une source lumineuse. L'éclairage permet avantageusement d'augmenter le contraste entre la particule et la solution.

L'invention permet, avantageusement, en utilisant une pluralité de particules, de détecter des détériorations faibles causées par l'action appliquée au film. L'utilisation d'une seule particule et/ou bille magnétique ou magnétisable ne permet pas de détecter ces détériorations En outre, il existe un risque non négligeable de détection de phénomènes non spécifiques, par exemple une formation partielle du film.

Selon l'invention, l'observation de peut être réalisée par tout moyen connue de l'homme du métier. Il peut s'agir par exemple d'un dispositif optique, par exemple un microscope, un appareil photo, d'un scanner de document, par exemple un scanner perfection V750 EPSON, une observation visuelle.

Selon l'invention, l'observation peut permettre de mesurer, par exemple l'intensité, le contraste, la variance, d'image, par exemple via tout moyen connu de l'homme du métier, par exemple un logiciel d'imagerie, par exemple ImageJ permettant, par exemple de mesurer, par exemple des différences de contrastes, d'intensités, correspondant par exemple aux particules, dans des zones d'une image à une autre et ainsi de déterminer les différences d'une observation à une autre. Il peut s'agir, par exemple de comparer les images obtenues avant et après application de l'action mécanique, hydrodynamique ou physique, par exemple en faisant une soustraction entre des images, par exemple en mesurant le coefficient de corrélation entre les images.

Selon l'invention, l'utilisation de particules émettrices d'un signal, par exemple des particules colorées, fluorescente, phosphorescentes, luminescentes, radioactives peut permettre, par exemple une observation automatisée

Selon l'invention, l'effet peut être déterminé par la visualisation de la répartition des particules. Par exemple, une zone sans particules peut être, par exemple une déchirure du film, la non dispersion des particules permet de déterminer la résistance du film à l'action,

Selon l'invention, le regroupement peut être réalisé avec tout moyen connu de l'homme du métier. Il peut s'agir par exemple lorsque les particules sont, par exemple magnétiques, magnétisables, d'un aimant, d'un champ électrique, électromagnétique permettant, par exemple de regroupe lesdites particules en un point.

Selon l'invention par point on entend, par exemple le regroupement à un endroit de la pluralité de particules formant ainsi sur la surface immergée un point, un disque, un anneau, une barre, une forme géométrique régulière, par exemple un carré, un losange, un triangle, ou en une tache,

Selon l'invention par tache on entend, par exemple une zone sombre formée par la pluralité de particule sur la surface immergée.

Selon l'invention, l'observation à l'étape (e) peut être effectuée sur une surface $S_1$ qui peut représenter la surface d'observation, par exemple sur une première image, par exemple sur une surface de 1 à 10'000, de 10 à 900 de 50 à 700, de 100 à 500 pixels.

Selon la présente invention la taille d'un pixel est définie par sa largeur $\times$ hauteur, par exemple la hauteur 0,018 à 0,660 mm, la largeur de 0,018 à 0,660 mm

Selon l'invention, l'observation à l'étape (g) peut être effectuée sur une surface $S_2$ qui peut représenter la surface d'observation, par exemple sur une seconde image, par exemple une surface égale à $S_1$, une surface de 1,1 à 2,5 fois celle de $S_1$, de 0,5 à 0,9 fois celle de $S_1$.

Avantageusement, la comparaison précitée peut permettre de mesurer le degré d'effet de l'action, par exemple elle peut permettre de mesurer le pourcentage et la quantité du film modifié par l'action.

Selon l'invention, la comparaison des observations peut permettre avantageusement de déterminer le pourcentage de dispersion des particules sur la surface immergée et ainsi une valeur de l'effet de l'action sur le film.

En outre le procédé de l'invention permet d'obtenir un résultat fiable, avec une meilleure sensibilité que les procédés de l'état de la technique.

En outre le procédé de l'invention permet de déterminer, sans réactif chimique ou biologique, l'impact de l'action sur le film et, de plus, de quantifier cet impact de manière reproductible, fidèle, dans un temps court et n'est pas dépendant d'un opérateur et/ou dispositif particulier.

Selon l'invention, le champ peut être appliqué dès le début et/ou au milieu de la formation du film.

Selon l'invention, le champ magnétique, ou électrique ou électromagnétique peut être tout champ permettant de mettre en mouvement lesdites, au moins deux particules sur ladite surface immergée dans ladite solution, par exemple un champ électromagnétique ou un champ magnétique. Le champ magnétique, ou électrique ou électromagnétique peut être généré, par exemple par un aimant ou par un solénoïde. L'aimant peut être par exemple sous forme de barreau, de pointe, de pièce, etc. ou toute forme appropriée pour la mise en oeuvre de la présente invention. Le champ peut, par exemple être appliqué par tout moyen connu de l'homme du métier, par exemple par impulsion, par augmentation progressive du champ électromagnétique, par variations de champ électromagnétique ou par une combinaison ces applications.

Selon l'invention, les particules peuvent être déposés sur la surface sous la forme d'une goutte contenant une substance liante soluble dans la solution ou qui peut se défaire lors de l'application d'une action.

Selon l'invention, le procédé permet également avantageusement de quantifier l'aspect des taches et/ou points observés après application de l'action.

Le procédé de l'invention peut comprendre après l'étape (f) une étape (i) de quantification (Q) de l'aspect des taches et/ou points par mesure de la déviation standard moyenne $D_1$. La détermination de la déviation standard moyenne peut être réalisée par tout procédé connu de l'homme du métier. La mesure peut être réalisée par exemple avec le logiciel ImageJ (Image Processing and Analysis in Java, http://rsb.info.nih.gov.ij), par exemple par mesure sur une image observée à l'étape (g) contenue, par exemple dans une ellipse centrée sur le point et/ou la tâche obtenu à l'étape (c), par le calcul, par de la valeur approchée de la variance de la forme du point ou de la tache observée à l'étape (g) égale à

$$Q=(D_1{}^*D_1 - D_0{}^*D_0)/(I{}^*I),$$

où I est la mesure du contraste, de l'intensité des taches telle que définie précédemment et $D_0$ est la déviation standard moyenne mesurée avec le logiciel ImageJ du fond du puits autour de la tache, par exemple dans une ellipse située au contact de la tache mais ne comprenant pas la tache elle-même.

Avantageusement, la présente invention permet de déterminer la formation, destruction de biofilms de microorganismes, de films formés, par exemple par des peintures, par des substances alimentaires, par des échantillons de milieux naturels ou industriels, par des échantillons biologiques, par exemple des biofilms de microorganismes qui peuvent être des film constitué par une ou plusieurs espèces de micro-organismes (bactéries, champignons, algues ou protozoaires), adhérant entre eux et à une surface, et sécrétant pas, peu ou en quantité variable une matrice adhésive et protectrice constituée généralement de polysaccharides.

En outre le procédé de l'invention permet d'obtenir un résultat fiable avec une meilleure sensibilité et une meilleure spécificité que les procédés de l'état de la technique.

De plus, le procédé de l'invention permet d'obtenir un résultat reproductible et donc permettant une comparaison efficace et utile des mesures effectuées avec le procédé de l'invention.

D'autres avantages pourront encore apparaître à l'homme du métier à la lecture des exemples ci-dessous, illustrés par les figures annexées, donnés à titre illustratif.

**Brève description des figures**

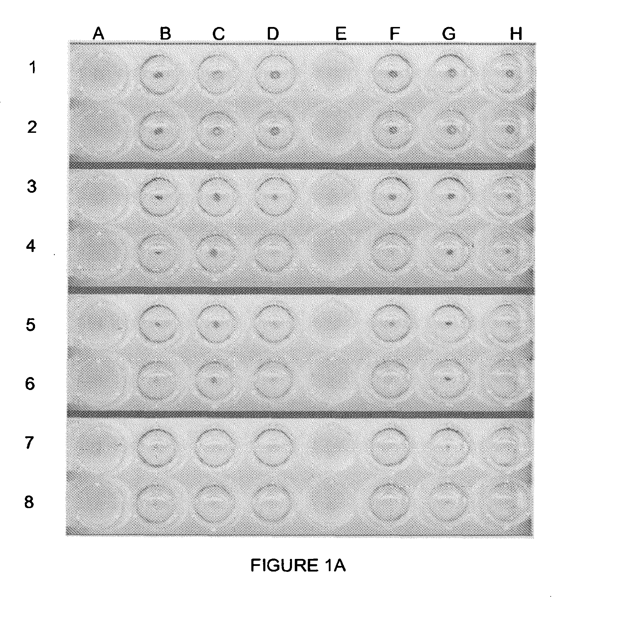

- La figure 1A représente une photographie de 6 barrettes indiquées de 1 à 6 comprenant respectivement dans les puits de la colonne A et E, de l'eau et des microbilles paramagnétiques, de la colonne B et F, du BHI à 37g/L et des microbilles paramagnétiques, de la colonne C et G, de la peptone à 5g/l et des microbilles paramagnétiques, de la colonne D et H, de la tryptone à 5g/L et des microbilles paramagnétiques, Les lignes correspondent à l'observation des puits après une incubation de 2h30 à 37°C, respectivement les lignes 1 et 2 avant agitation, les lignes 3 et 4 après 20 secondes d'agitation, les lignes 5 et 6 après 60 secondes d'agitation, les lignes 7 et 8 après 240 secondes d'agitation,

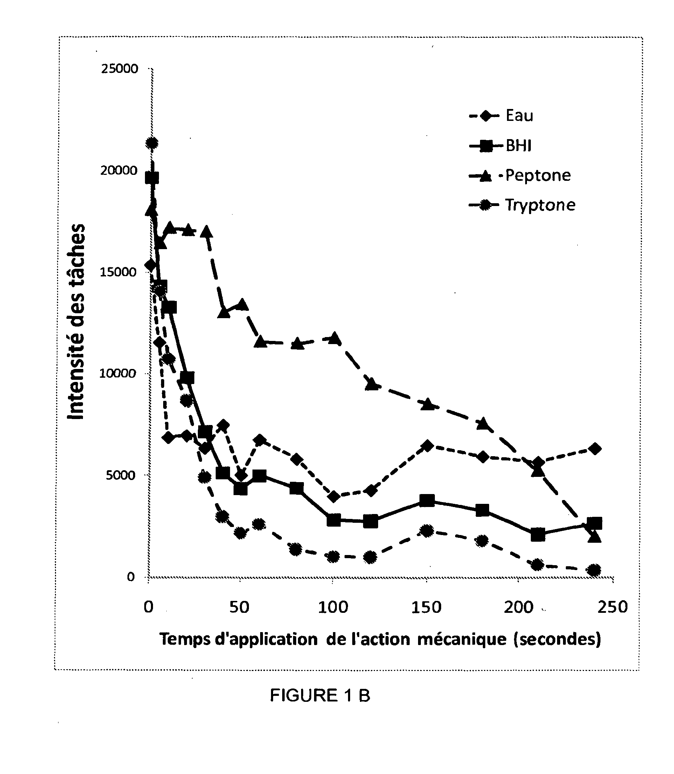

- La figure 1B représente un schéma de la variation de l'intensité attribuable (Iatt) des taches (en abscisse) en fonction du temps (T) d'action hydrodynamique en secondes (ordonnée). Les carrées plein représentent les valeurs obtenues pour les puits comprenant du milieu coeur cervelle infusion (ou Brain Heart Infusion appelé communément BHI), les triangles pleins représentent les valeurs obtenues pour les puits comprenant de la peptone, les ronds pleins représentent les valeurs obtenues pour les puits comprenant de la tryptone et les losanges pleins représentent les valeurs obtenues pour les puits comprenant de l'eau.

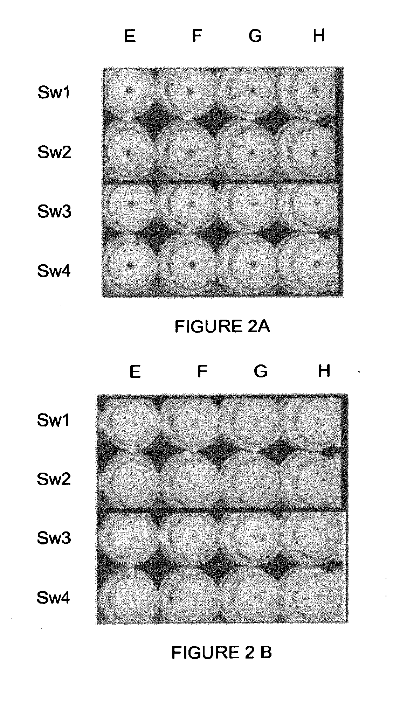

- Les figures 2A et 2B sont des photographies de puits de barrettes.

- La figure 2A représente une photographie de 4 barrettes indiquées sw 1 à sw 4 comprenant respectivement les bactéries suivantes : *Listeria monocytogenes, Escherichia coli* DH5 α, *Staphylococcus xylosus, Staphylococus carnosus* et des particules magnétique avant action hydrodynamique.

- La figure 2B représente une photographie de 4 barrettes indiquées sw 1 à sw 4 comprenant respectivement les bactéries suivantes : *Listeria monocytogenes, Escherichia coli* DH5 α, *Staphylococcus xylosus, Staphylococus carnosus* et des particules magnétique après action hydrodynamique.

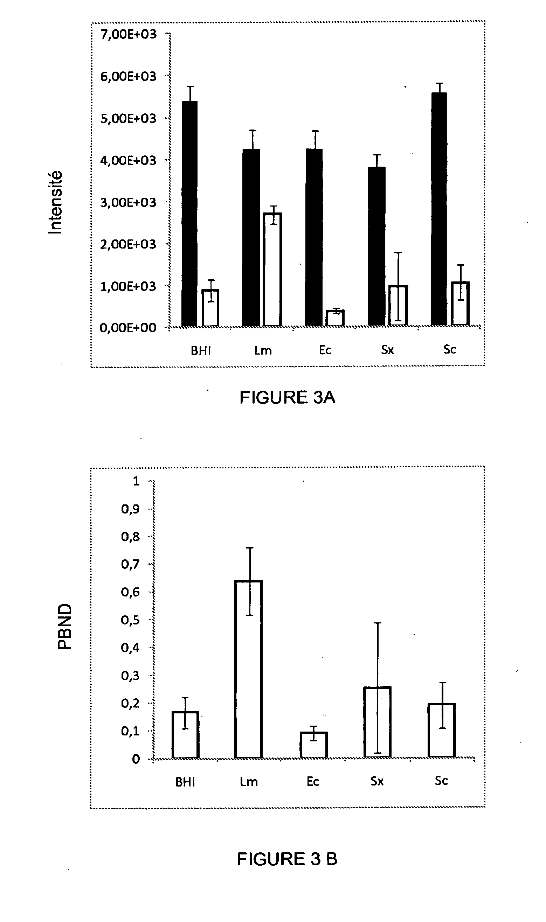

- La figure 3A est un diagramme en bâtons représentant les résultats de l'intensité (I) (ordonnée) en fonction de la bactérie (abscisse), BHI représente les puits sans bactéries, Lm : *Listeria monocytogenes,* Ec : *Escherichia coli* DH5 α, Sx : *Staphylococcus xylosus,* Sc : *Staphylococus carnosus.* Bâtons en noir : valeurs avant agitation, bâtons en blanc : valeurs après agitation.

- La figure 3B est un diagramme en bâtons représentant une normalisation des valeurs obtenues en 3 A, correspondant à la Proportion de Biofilm Non Défait (PBND) après l'action hydrodynamique (ordonnée) en fonction de la bactérie (abscisse). BHI représente les puits sans bactéries, Lm : *Listeria monocytogenes,* Ec : *Escherichia coli* DH5 α, Sx : *Staphylococcus xylosus,* Sc : *Staphylococus carnosus.*

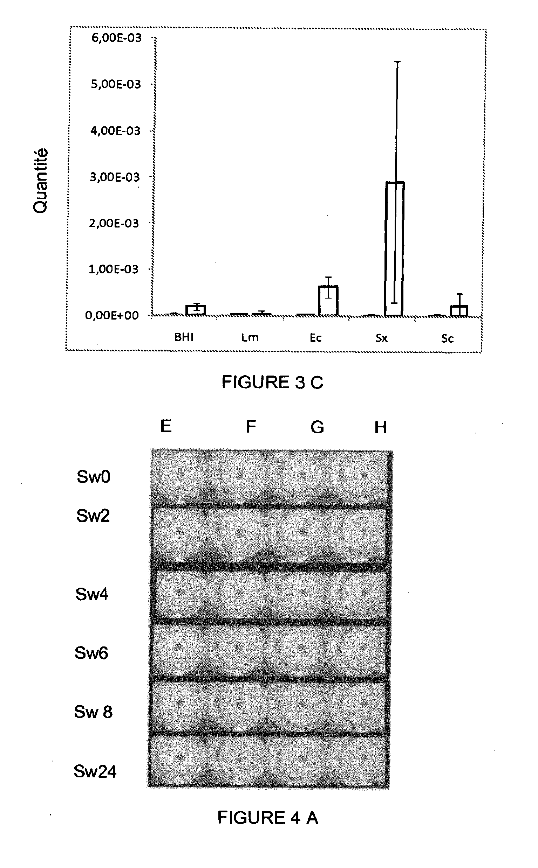

- La figure 3C est un diagramme en bâtons représentant la quantification Q (ordonnée) en fonction des bactéries. BHI représente les puits sans bactéries, Lm : *Listeria monocytogenes,* Ec : *Escherichia coli* DH5 α, Sx : *Staphylococcus xylosus,* Sc : *Staphylococus carnosus.*

- La figure 4A représente une photographie de 6 barrettes indiquées sw 0, sw 2, sw 4, sw 6, sw 8 et sw 24 (respectivement après 0, 2, 4, 6, 8, et 24 heures de culture à 37°C) comprenant dans le puits E du milieu BHI et des particules magnétiques, et dans les puits F, G et H les bactéries *Listeria monocytogenes* dans du milieu BHI avec des particules magnétiques avant action hydrodynamique.

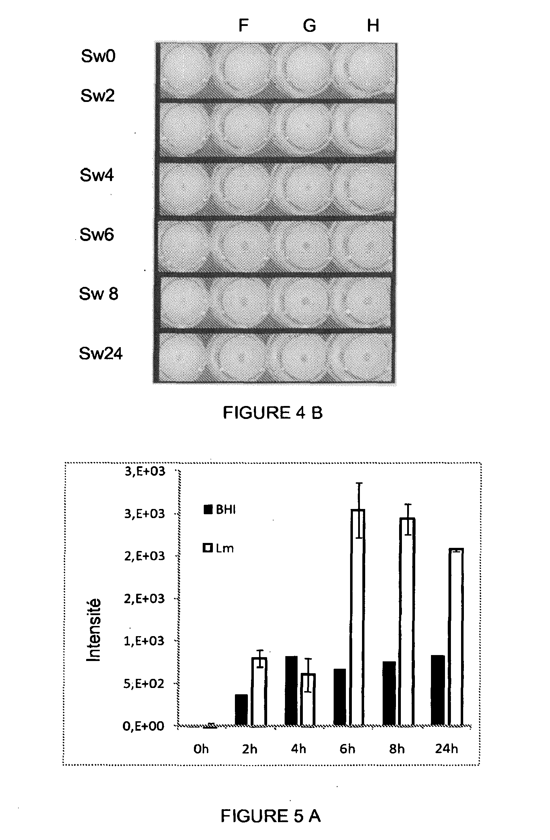

- La figure 4B représente une photographie de 6 barrettes indiquées sw 0, sw 2, sw 4, sw 6, sw 8 et sw 24 (respectivement après 0, 2, 4, 6, 8, et 24 heures de culture à 37°C) comprenant dans le puits E du milieu BHI et des particules magnétiques, et dans les puits F, G et H les bactéries *Listeria monocytogenes* dans du milieu BHI avec des particules magnétiques après action hydrodynamique.

- La figure 5A est un est un diagramme en bâton représentant les résultats de l'intensité (I) (ordonnée) en fonction du temps (abscisse) à 0, 2, 4, 6, 8 ou 24 heures. Les bâtons vides correspondent aux résultats obtenus avec *Listeria monocytogenes (Lm),* les bâtons noirs aux résultats sans bactéries (BHI).

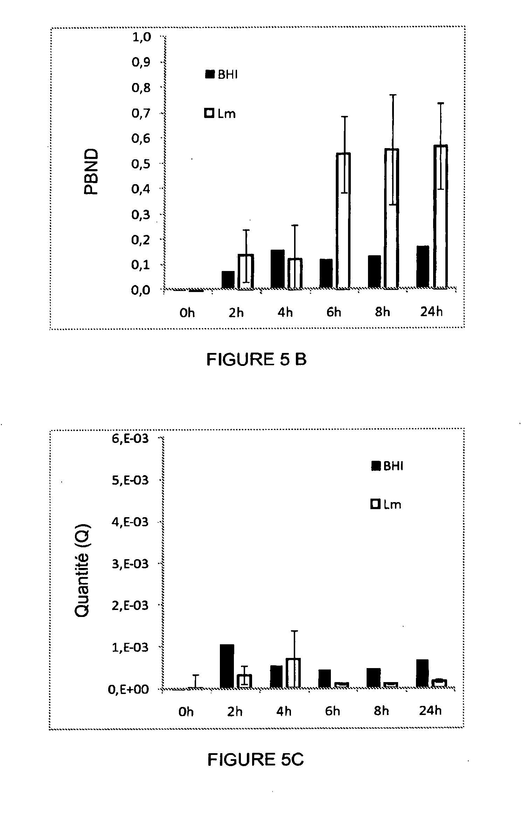

- La figure 5B est un diagramme en bâton représentant la proportion de Biofilm Non Défait (PBND) après l'action hydrodynamique (ordonnée) en fonction du temps (abscisse) à 0, 2, 4, 6, 8 ou 24 heures. Les bâtons vides correspondent aux résultats obtenus avec *Listeria monocytogenes (Lm),* les bâtons noirs au résultat sans bactéries (BHI).

- La figure 5C est un diagramme en bâton représentant la quantification Q (ordonnée) en fonction du temps (abscisse) à 0, 2, 4, 6, 8 ou 24 heures. Les bâtons vides correspondent aux résultats obtenus avec *Listeria monocytogenes (Lm),* les bâtons noirs aux résultats sans bactéries (BHI).

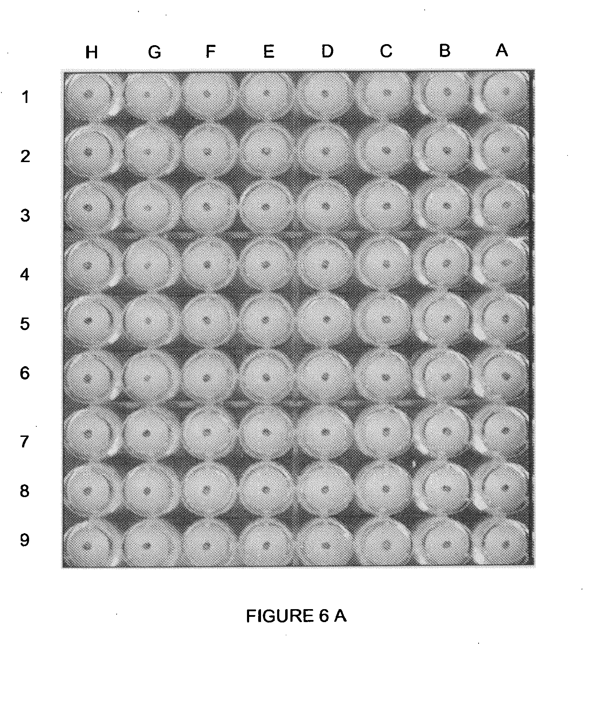

- La figure 6A représente une photographie de 9 barrettes de 8 puits indiquées de 1 à 9 comprenant des bactéries *Staphylococcus aureus CIP 76.25A* (ensemencées dans le milieu de culture des puits dans les colonne B, C, D, E, F et G) comprenant dans le milieu de culture respectivement de l'Ampicilline (ligne 1), de la Ceftazidime (ligne 2), du Chloramphenicol (ligne 3), de l'Erythromicine (ligne 4), de la Piperacilline (ligne 5), de la Tetracycline (ligne 6), de la Gentamicine (ligne 7), de la Ciprofloxacine (ligne 8) ou de la Trimethoprime (ligne 9), en fonction de la concentration : sans antibiotiques (colonne A = témoin de stérilité du milieu de culture avec particules magnétiques, sans bactéries, et colonne G = témoin milieu de culture avec bactéries, avec particules magnétiques), 4 fois la concentration de base de l'antibiotique (colonnes F et H), 2 fois la concentration de base de l'antibiotique (colonne G), concentration de base de l'antibiotique (colonne D), 0,5 fois la concentration de base de l'antibiotique (colonne C) ou 0,25 fois la concentration de base de l'antibiotique (colonne B) et des particules magnétiques avant action hydrodynamique.

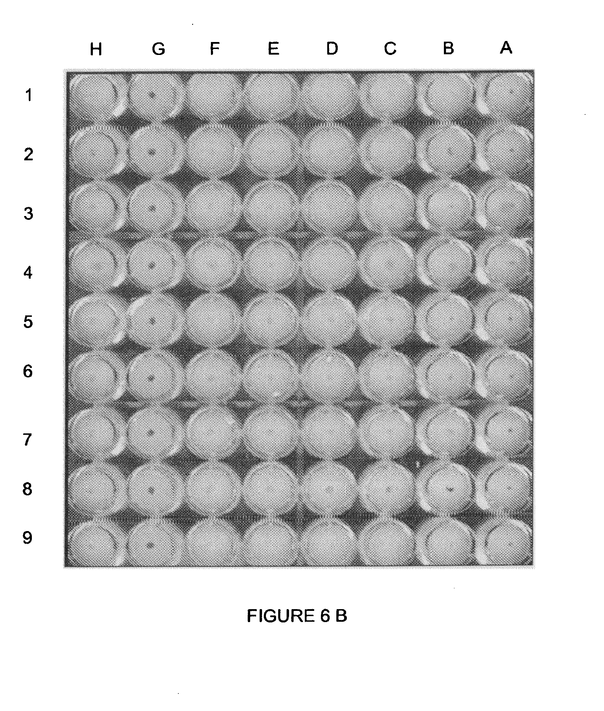

- La figure 6B représente la configuration de la figure 6A mais après action hydrodynamique.

## EXEMPLES

**Exemple 1 : mesure de l'effet d'une action hydrodynamique sur des films de protéines.**

Dans cet exemple la méthode est appliquée à l'étude de la résistance de films formés à partir de solutions riches en protéines.

Il a été préparé 2,4 ml de solutions, respectivement, d'eau, de BHI (BD-DIFCO, France) à 37g/l, de peptone (Fluka) à 5g/l et de Bacto (marque déposée) Tryptone (BD-DIFCO, France) à 5g/l. Ces solutions ont été supplémentées avec une solution de microbilles paramagnétiques (Ton006N, BiofilmControl, France) à raison de 10$\mu$l/ml. Ces solutions ont été alors déposées à raison de 100$\mu$l par puits, dans les puits, respectivement, A et E, B et F, C et G, D et H, de 2 barrettes de puits à fond plat (Référence : MSW002B, BioFilm Control, France). Les barrettes ont été placées sur des blocs tests aimantés (BKT-MSW002 BioFilm Control, France), le tout a été placé dans une étuve thermostatique (Référence BC240, Firelabo, France) stabilisée à 37°C pendant 2h30.

Les barrettes ont été ensuite placées sur un agitateur orbital (Variomag Monoshake, H+P Labortechnik, Allemagne) ajusté à 30% +/-10% de sa vitesse de rotation maximale. pour les soumettre à une action hydrodynamique, successivement pendant, 0s, 5s, 5s, 10s, 10s, 10s, 10s, 10,s, 20s, 20s, 20s, 30s, 30s, 30s, 30, totalisant ainsi des temps d'agitation totale de 0s, 5s, 10s, 20s, 30s, 40s, 50s, 60s, 80s, 100s, 120s, 150s, 180s, 210s, et 240s.

Après chaque action d'agitation, les barrettes ont été placées sur un scanner de documents (perfection V-750 PRO, Epson, USA) avec lequel une prise d'image a été effectuée avec le logiciel EpsonScan (Epson, USA).

Les images reproduites en figure 1A pour des durées d'agitation de 0s, 20s, 60s et 240s ont été obtenues en additionnant les composantes rouges vertes et bleus des images couleurs obtenues avec le scanner en utilisant le logiciel ImageJ (http://rsb.info.nih.gov.ij) et un découpage des images obtenues avec différents réglages du contraste. Des points bien définis sont visibles dans tous les puits.

Les points et tâches ont été quantifiées en réalisant deux mesures, respectivement $I_1$ et $I_2$, de l'intensité moyenne de l'image contenu dans une ellipse centrée sur le point ou la tâche de surface, respectivement, $S_1$ = 100 à 500 pixels et $S_2$ = 1,1x$S_1$ à 2,5x$S_1$, avec le logiciel ImageJ.

Une mesure approchée de l'intensité attribuable ($I_{att}$) au point ou à la tache sombre observée dans les images est obtenue en effectuant le calcul suivant :

$$I_{att} = S_1 \times (I_2 \times S_2 - I_1 \times S_1) / (S_2 - S_1) - I_1 \times S_1$$

Les valeurs moyennes des intensités ainsi obtenues sont reproduites figure 1B et dans le tableau 1 ci-dessous.

Tableau 1 de résultats de mesure de l'intensité mesurée

| durée d'application de l'action hydrodynamique en seconde | Intensité des taches | | | |
|---|---|---|---|---|
| | Eau | BHI | Peptone | Tryptone |
| 0 | 15356 | 19650 | 18068 | 21335 |
| 5 | 11538 | 14306 | 16421 | 14074 |
| 10 | 6878 | 13273 | 17179 | 10715 |
| 20 | 6975 | 9803 | 17081 | 8701 |
| 30 | 6361 | 7180 | 16995 | 4907 |
| 40 | 7494 | 5137 | 13027 | 2978 |
| 50 | 5031 | 4368 | 13421 | 2176 |

(suite)

| durée d'application de l'action hydrodynamique en seconde | Intensité des taches | | | |
|---|---|---|---|---|
| | Eau | BHI | Peptone | Tryptone |
| 60 | 6772 | 5012 | 11587 | 2627 |
| 80 | 5823 | 4391 | 11498 | 1378 |
| 100 | 3983 | 2825 | 11777 | 1017 |
| 120 | 4287 | 2771 | 9509 | 993 |
| 150 | 6495 | 3777 | 8523 | 2285 |
| 180 | 5942 | 3304 | 7575 | 1799 |
| 210 | 5668 | 2114 | 5242 | 607 |
| 240 | 6338 | 2644 | 1993 | 337 |

Comme représenté sur la figure 1B et dans le tableau cidessus, l'intensité d'image varie en fonction du temps d'agitation et permet de mesurer, par exemple la résistance de films. Comme démontré dans cet exemple, le procédé de l'invention permet de déterminer l'effet d'une action, par exemple une action hydrodynamique sur un film. En particulier, le procédé de l'invention permet de déterminer la résistance de films les uns par rapport aux autres.

**Exemple 2 : mesure de l'effet d'une action hydrodynamique sur des biofilms de différentes espèces.**

Dans cet exemple le procédé de l'invention permet d'étudier la résistance de biofilms formés par différentes espèces bactériennes.

Des cultures de 16 heures en milieu coeur cervelle infusion (BHI, BD-DIFCO (France)) des 4 microorganismes suivants : *Listeria monocytogenes, Escherichia coli* DH5 $\alpha$, *Staphylococcus xylosus, Staphylococus carnosus* ont été ajustées à une densité optique à une longueur d'onde de 600 nm $DO_{600nm}$=0,004 par dilution avec du BHI stérile, supplémentées avec une solution de microbilles paramagnétiques (Ton005N, BioFilmControl, France) à une concentration de 10$\mu$l/ml. La mesure de la densité optique a été réalisée avec un spectrophotomètre (Biomate, Thermo Scientific, France). La culture ajustée comprenant les microbilles paramagnétique a été déposée dans les puits à fond plat (Référence : MSW002B, BioFilm Control, France) à raison de 200$\mu$l/puits dans les puits référencés F, G et H de 4 barrettes, référencées respectivement sw1, sw2, sw3 et sw4. 200$\mu$l de BHI stérile supplémentées avec une solution de microbilles paramagnétiques (Ton005N, BioFilmControl, France) à raison de 10$\mu$l/ml ont été déposées dans les puits E de chacune des barrettes.

La répartition des différents dépôts est représentée dans le tableau 2 ci-dessous.

Tableau 2

| | E | F | G | H |
|---|---|---|---|---|
| Sw1 | *BHI stérile* | *Listeria monocytogenes* | *Listeria monocytogenes* | *Listeria monocytogenes* |
| Sw2 | *BHI stérile* | *Escherichia coli* DH5 $\alpha$ | *Escherichia coli* DH5 $\alpha$ | *Escherichia coli* DH5 $\alpha$ |
| Sw3 | *BHI stérile* | *Staphylococcus xylosus* | *Staphylococcus xylosus* | *Staphylococcus xylosus* |
| Sw4 | *BHI stérile* | *Staphylococus carnosus* | *Staphylococcus carnosus* | *Staphylococus carnosus* |

Chaque barrette a été placée indépendamment sur un bloc test aimanté (BKT-MSW002 BioFilm Control, France) placé dans des boîtes rectangulaires avec couvercle de 18x12x7cm contenant deux béchers de 25ml contenant 20 ml d'eau. L'ensemble a été ensuite placé dans une étuve thermostatique (Référence BC240, Firelabo, France) stabilisée à 30°C pour les barrettes sw1 et sw2 et stabilisée à 37°C pour les barrettes sw3 et sw4 pendant 8 heures.

Les barrettes ont été ensuite placées indépendamment sur un scanner de documents (perfection V-750 PRO, Epson, USA) avec lequel une prise d'image a été effectuée avec le logiciel Epson Scan (Epson, USA). Les images finales reproduites en figure 2A ont été obtenues en additionnant les composantes rouges vertes et bleus des images couleurs obtenues avec le scanner en utilisant le logiciel ImageJ (Image Processing and Analysis in Java, http://rsb.info.nih.gov.ij) et un découpage des images obtenues avec différents réglages du contraste. Des points bien définis sont visibles dans tous les puits.

Les barrettes sont ensuite placées sur un agitateur orbital (Variomag Monoshake, H+P Labortechnik, Allemagne) ajusté à 60% +/-20% de sa vitesse de rotation maximale pendant 10 secondes. Cette étape correspond à la soumission du film formé dans la culture à une action hydrodynamique.

Une seconde prise d'images a été effectuée dans les mêmes conditions que la première image. L'image ainsi obtenu correspond à la figure 2B. Sur cette figure, des points et tâches plus ou moins bien définies sont visibles, et leur aspect dépend de la souche bactérienne ayant formé le biofilm. Les points en ligne E sont nettement moins intenses, ce qui correspond à une dispersion maximale des microbilles paramagnétiques sous l'action hydrodynamique.

Les points et tâches ont été quantifiées en réalisant deux mesures, respectivement $I_1$ et $I_2$, de l'intensité moyenne de l'image contenu dans une ellipse centrée sur le point ou la tâche de surface, respectivement, S1 = 100 à 500 pixels et S2 = 1,1xS1 à 2,5xS1, avec le logiciel ImageJ.

Une mesure approchée de l'intensité attribuable ($I_{att}$) au point ou à la tache sombre observée dans les images est obtenue en effectuant le calcul suivant :

$$I_{att} = S_1 \times (I_2 \times S_2 - I_1 \times S_1) / (S_2 - S_1) - I_1 \times S_1$$

Les valeurs moyennes des intensités ainsi obtenues sont reproduites figure 3A et dans le tableau 3 ci-dessous, les barres d'erreur correspondant à l'écart type des mesures effectuées.

Tableau 3 : résultats de mesure de l'intensité mesurée

| Iatt | Avant Magnétisation | | Après magnétisation | |
|---|---|---|---|---|
| | Moyenne | Ecart type | Moyenne | Ecart type |
| BHI | 5,40E+03 | 3,45E+02 | 8,86E+02 | 2,49E+02 |
| Lm | 4,24E+03 | 4,72E+02 | 2,69E+03 | 2,16E+02 |
| Ec | 4,24E+03 | 4,50E+02 | 3,80E+02 | 7,41E+01 |
| Sx | 3,82E+03 | 3,03E+02 | 9,49E+02 | 8,20E+02 |
| Sc | 5,57E+03 | 22,30E+02 | 1,04E+03 | 4,23E+02 |

Les intensités brutes après magnétisation sont normées en les divisant par l'intensité brutes avant magnétisation et permet d'obtenir une mesure approchée de la proportion de biofilm non défait par l'action hydrodynamique (PBND). Les résultats obtenus pour cet exemple sont reproduits dans la figure 3B et dans le tableau 4 ci-dessous, les barres d'erreurs correspondant à l'écart type de mesures.

Tableau 4

| milieu | Moyenne | Ecart type |
|---|---|---|
| BHI | 0,16393578 | 0,05648906 |
| Lm | 0,63556509 | 0,12167536 |
| Ec | 0,08963821 | 0,0269836 |
| Sx | 0,24868618 | 0,2345908 |
| Sc | 0,1874682 | 0,08374593 |

L'aspect des points et tâches peuvent également être quantifiées (valeur Q) en la déduisant de la déviation standard moyenne D1 mesurée avec le logiciel ImageJ de l'image contenue dans une ellipse centrée sur le point ou la tâche par le calcul de la valeur approchée de la variance de la forme du point ou de la tache égale à

$$Q = (D1*D1 - D0*D0) / (I*I),$$

où I est l'intensité calculée comme décrit plus haut et D0 est la déviation standard moyenne mesurée avec le logiciel ImageJ du fond du point autour de la tache.

EP 2 655 652 B1

Les résultats obtenus pour cet exemple sont reproduits dans la figure 3C dans le tableau 5 ci-dessous, les barres d'erreur correspondant à l'écart type de mesures.

Tableau 5

| variances normées | Avant Magnétisation | | Après magnétisation | |
|---|---|---|---|---|
| | Moyenne | Ecart type | Moyenne | Ecart type |
| BHI | 1.88E-04 | 7.63E-05 | 3,38E-05 | 8,57E-06 |
| Lm | 1,98E-05 | 9,43E-05 | 3,54E-05 | 1,11E-05 |
| Ec | 6,33E-04 | 2,29E-04 | 3,97E-05 | 7,11E-06 |
| Sx | 2,91E-03 | 2,61E-03 | 2,80E-05 | 9,39E-06 |
| Sc | 2,17E-04 | 2,98E-04 | 2,84E-05 | 8,56E-06 |

Comme démontré dans cet exemple, le procédé de l'invention permet de déterminer l'effet d'une action, par exemple une action hydrodynamique sur un biofilm. En particulier, le procédé de l'invention permet de déterminer la résistance de films les uns par rapport aux autres.

En outre, le procédé de l'invention permet de détecter de manière très sensible et rapide la déviation des particules et l'effet de l'action sur le film.

**Exemple 3 : mesure de l'effet d'une action hydrodynamique sur des biofilms en cours de formation.**

Dans cet exemple les dispositifs et produits utilisés sont identiques à ceux de l'exemple précédent.

Une culture de 16 heures en milieu BHI (BD-DIFCO (France)) de *Listeria monocytogenes,* est ajustée à DO$_{600nm}$=0,004 par dilution avec du BHI stérile, supplémentées avec une solution de microbilles paramagnétiques (Ton005N, BiofilmControl, France) à raison de 10$\mu$l/ml, puis 200$\mu$l/puits a été déposés dans les puits à fond plat (Référence : MSW002B, BioFilm Control, France) indiqués F, G et H de des 6 barrettes, respectivement, sw0, sw2, sw4, sw6, sw8 et sw24. 200$\mu$l de BHI stérile supplémentées avec 10$\mu$l/ml d'une solution de microbilles paramagnétiques (Ton005N, BioFilmControl, France) ont été déposées dans les puits E de chacune des barrettes.

La répartition des différents dépôts est représentée dans le tableau 6 ci-dessous.

Tableau 6

| | E | F | G | G |
|---|---|---|---|---|
| Sw0 | *BHI stérile* | *Listeria monocytogenes* | *Listeria monocytogenes* | *Listeria monocytogenes* |
| Sw2 | *BHI stérile* | *Listeria monocytogenes* | *Listeria monocytogenes* | *Listeria monocytogenes* |
| Sw4 | *BHI stérile* | *Listeria monocytogenes* | *Listeria monocytogenes* | *Listeria monocytogenes* |
| Sw8 | *BHI stérile* | *Listeria monocytogenes* | *Listeria monocytogenes* | *Listeria monocytogenes* |
| Sw24 | *BHI stérile* | *Listeria monocytogenes* | *Listeria monocytogenes* | *Listeria monocytogenes* |

Les barrettes ont été placées sur des bloc test aimanté (BKT-MSW002 BioFilm Control, France) placés dans des boîtes rectangulaires avec couvercle de 18x12x7cm contenant deux béchers de 25ml contenant 20 ml d'eau, le tout étant placé dans une étuve thermostatique (Référence BC240, Firelabo, France) stabilisée à 30°C pendant, respectivement, 0h, 2h, 4h, 6h, 8h et 24h.

Les barrettes ont été ensuite placées sur un scanner de documents (perfection V-750 PRO, Epson, USA) avec lequel une prise d'image a été effectuée avec le logiciel EpsonScan (Epson, USA). Les images finales reproduites en figure 4A ont été obtenues en additionnant les composantes rouges vertes et bleus des images de la composante rouge des images couleurs obtenues avec le scanner en utilisant le logiciel ImageJ (http://rsb.info.nih.gov.ij) et un découpage des images obtenues avec différents réglages du contraste. L'image obtenue est représentée sur la figure 4A. Des points bien définis sont visibles dans tous les puits.

Les barrettes ont été ensuite placées sur un agitateur orbital (Variomag Monoshake, H+P Labortechnik, Allemagne) ajusté à 60% +/-20% de sa vitesse de rotation maximale pendant 10 secondes pour les soumettre à une action hydrodynamique.

Une seconde prise d'images à été effectuée dans les mêmes conditions que la première image et l'image finale est reproduite dans la figure 4B. Des points et tâches plus ou moins bien définies sont visibles, et leur aspect dépend de la souche bactérienne ayant formé le biofilm.

Les points et tâches ont été quantifiés en réalisant deux mesures avec le logiciel ImageJ, respectivement $I_1$ et $I_2$, de l'intensité moyenne de l'image contenu dans une ellipse centrée sur le point ou la tâche de surface, respectivement.

S1 = 100 à 500 pixels et S2 = 1.1xS1 à 2.5xS1.

Une mesure approchée de l'intensité attribuable ($I_{att}$) au point ou à la tache sombre observée dans les images est obtenue en effectuant le calcul suivant :

$$I_{att}= S_1 \times (I_2 \times S_2 - I_1 \times S_1) / (S_2 - S_1) - I_1 \times S_1$$

Les valeurs moyennes des intensités ainsi obtenues sont reproduites figure 5A et dans le tableau 7 ci-dessous., les barres d'erreur correspondant à l'écart type des mesures effectuées.

Tableau 7

| temps | BHI | Lm | Ecart type Lm |
|---|---|---|---|
| 0h | -1,72E+01 | -5.82E+00 | 3,55E+01 |
| 2h | 3.74E+02 | 7,94E+02 | 9,81E+01 |
| 4h | 18,14E+02 | 6,02E+02 | 1,95E+02 |
| 6h | 6,64E+02 | 2,54E+03 | 3,29E+02 |
| 8h | 7,52E+02 | 2,44E+03 | 1,79E+02 |
| 24h | 8,26E+02 | 2,07E+03 | 2,19E+01 |

Les intensités brutes après magnétisation ont été normées en les divisant par l'intensité brutes avant magnétisation et permet d'obtenir une mesure approché de la proportion de biofilm non défait (PBND) par l'action hydrodynamique. Ces résultats sont reproduit en figure 5B et dans le tableau 8 ci-dessous.

Tableau 8

| temps | BHI | Lm | Ecart type Lm |
|---|---|---|---|
| 0h | -0,00360422 | -0,0011256 | 1,04E-03 |
| 2h | 0,06975705 | 0,13379324 | 1,05E-01 |
| 4h | 0,15521974 | 0,11969938 | 1,34E-01 |
| 6h | 0,11833857 | 0,533572 | 1,52E-01 |
| 8h | 0,1325017 | 0,5516224 | 2.17E-01 |
| 24h | 0,16997323 | 0,5647522 | 1,72E-01 |

L'aspect des points et tâches peuvent également être quantifiées Q en la déduisant de la déviation standard moyenne D1 mesurée avec le logiciel ImageJ de l'image contenue dans une ellipse centrée sur le point ou la tâche par le calcul de la valeur approchée de la variance de la forme du point ou de la tache selon la formule suivante :

$$Q = (D1*D1 - D0*D0)/(I*I),$$

où I est l'intensité calculée comme décrit plus haut et D0 est la déviation standard moyenne mesurée avec le logiciel ImageJ du fond du point autour de la tache ou du spot.

Les résultats obtenus pour cet exemple sont reproduits dans la figure 5C, les barres d'erreur correspondant à l'écart type de mesures.

Tableau 9

| temps | BHI | Lm | Ecart type Lm |
|---|---|---|---|
| 0h | -4,25E-03 | -3,30E-03 | 0,003635176 |
| 2h | 1,06E-03 | 3,20E-04 | 0,000216958 |
| 4h | 5,36E-04 | 6,81E-04 | 0,000686718 |
| 6h | 4.35E-04 | 9,43E-05 | 1,33109E-05 |
| 8h | 4,63E-04 | 1,08E-04 | 3,16769E-05 |
| 24h | 6,71E-04 | 1,64E-04 | 3,72707E-05 |

Comme démontré dans cet exemple, le procédé de l'invention permet de déterminer l'effet d'une action, par exemple une action hydrodynamique sur un biofilm. En particulier, le procédé de l'invention permet de déterminer la résistance de films les uns par rapport aux autres.

En outre, le procédé de l'invention permet de détecter de manière très sensible et rapide la déviation des particules et l'effet de l'action sur le film.

**Exemple 4: mesure de l'effet d'un antibiotique et d'une action hydrodynamique sur des biofilms.**

Dans cet exemple les dispositifs et produits utilisés sont identiques à ceux de l'exemple précédent.

Une culture de 16 heures en milieu BHI (BD-DIFCO (France)) de *Staphylococcus aureus CIP 76.25A* est ajustée à $DO_{600nm}$=0,004 par dilution avec du BHI stérile, supplémentées avec 10$\mu$l/ml d'une solution de microbilles paramagnétiques (Ton005N, BioFilmControl, France) et 200$\mu$l/puits a été déposés dans les puits à fond plat, respectivement lignes 1 à 9, et respectivement ; colonnes G à B, d'une plaque (Référence : MMB002B BioFilm Control, France). Chaque solution a été supplémentée avec des antibiotiques, respectivement, Ampicilline C=0,5 $\mu$g/mL, Ceftazidime C=16$\mu$g/mL, Chloramphenicol C=16$\mu$g/mL, Erythromicine C=0,5$\mu$g/mL, Piperacilline C=1$\mu$g/mL, Tetracycline C=2$\mu$g/mL, Gentamicine C=16$\mu$g/mL, Ciprofloxacine C=2$\mu$g/mL, Trimethoprime C=64$\mu$g/mL, à différentes concentrations, respectivement, 0xC, 4xC, 2xC, C, 0,5xC, 0,25xC.

Dans les puits de la colonne H, lignes respectivement 1à 9, ont été déposés 200$\mu$l de BHI supplémentées avec 10$\mu$l/ml d'une solution de microbilles paramagnétiques (Ton005N, BioFilmControl, France) et avec de l'antibiotique à une concentration de 4xC de, respectivement, Ampicilline C=0,5 $\mu$g/mL, Ceftazidime C=16$\mu$g/mL, Chloramphenicol C=16$\mu$g/mL, Erythromicine C=0,5$\mu$g/mL, Piperacilline C=1$\mu$g/mL, Tetracycline C=2$\mu$g/mL, Gentamicine C=16$\mu$g/mL, Ciprofloxacine C=2$\mu$g/mL, Trimethoprime C=64$\mu$g/mL.

La colonne A correspond à des puits témoins BHI stérile supplémenté uniquement avec une solution de microbilles paramagnétiques.

La colonne G correspond à des témoins de viabilité de *Staphylococcus aureus CIP 76.25A.* La colonne H correspond à des puits témoins BHI stérile supplémenté avec une solution de microbilles paramagnétiques en présence d'une dose maximale en antibiotiques.

La répartition des différents dépôts est représentée dans le tableau 10 ci-dessous.

Tableau 10

| | H | G | F | E | D | C | B | A |
|---|---|---|---|---|---|---|---|---|
| *Staphylococcus aureus* | - | + | + | + | + | + | + | - |
| 1 Ampicilline | 4xC | 0xC | 4xC | 2xC | C | 0,5xC | 0,25xC | 0xC |
| 2 Ceftazidime | 4xC | 0xC | 4xC | 2xC | C | 0,5xC | 0,25xC | 0xC |
| 3 Chloramphenicol | 4xC | 0xC | 4xC | 2xC | C | 0,5xC | 0,25xC | 0xC |
| 4 Erythromicine | 4xC | 0xC | 4xC | 2xC | C | 0,5xC | 0,25xC | 0xC |
| 5 Piperacilline | 4xC | 0xC | 4xC | 2xC | C | 0,5xC | 0,25xC | 0xC |
| 6 Tetracycline | 4xC | 0xC | 4xC | 2xC | C | 0,5xC | 0,25xC | 0xC |
| 7 Gentamicine | 4xC | 0xC | 4xC | 2xC | C | 0,5xC | 0,25xC | 0xC |
| 8 Ciprofloxacine | 4xC | 0xC | 4xC | 2xC | C | 0,5xC | 0,25xC | 0xC |

(suite)

| | H | G | F | E | D | C | B | A |
|---|---|---|---|---|---|---|---|---|
| 9 Trimethoprime | 4xC | 0xC | 4xC | 2xC | C | 0,5xC | 0,25xC | 0xC |

Les barrettes ont été placées sur des blocs tests aimantés (BKT-MSW002 BioFilm Control, France) placés dans des boîtes rectangulaires avec couvercle de 18x12x7cm contenant deux béchers de 25ml contenant 20 ml d'eau. L'ensemble a été placé dans une étuve thermostatique (Référence BC240, Firelabo, France) stabilisée à 37°C pendant 16 heures.

Les barrettes ont été ensuite placées sur un scanner de documents (perfection V-750 PRO, Epson, USA) avec lequel une prise d'image a été effectuée avec le logiciel EpsonScan (Epson, USA).

Les images finales obtenues sont représentées sur la figure 6A. Elles ont été obtenues en additionnant les composantes rouges vertes et bleus des images de la composante rouge des images couleurs obtenues avec le scanner en utilisant le logiciel ImageJ (http://rsb.info.nih.gov.ij) et un découpage des images obtenues avec différents réglages du contraste.

Comme représenté sur la figure 6A, les points sont bien définis et sont visibles dans tous les puits.

Les barrettes ont été ensuite placées sur un agitateur orbital (Variomag Monoshake, H+P Labortechnik, Allemagne) ajusté à 60% +/-20% de sa vitesse de rotation maximale pendant 10 secondes pour les soumettre à une action hydrodynamique.

Une seconde prise d'images à été effectué dans les mêmes conditions que la première image et l'image finale obtenue est représenté sur la figure 6B.

Comme représenté sur la figure 6B, des points et tâches plus ou moins bien définies sont visibles, et leur aspect dépend de l'antibiotique et de la concentration d'antibiotique utilisée.

Comme démontré dans cet exemple, le procédé de l'invention permet de déterminer l'effet d'une action, par exemple une action hydrodynamique sur un biofilm. En particulier, le procédé de l'invention permet de déterminer la résistance de films les uns par rapport aux autres en présence ou en absence d'agents

**Listes des références**

1. Physica Scripta. Vol. 65, 167-180 (2002) : « Refractive Index Measurement and its Applications »

2. Vörös et al. Biophysical Journal, Vol. 87 , 553-561 (2004) : « The Density and Refractive Index of Adsorbing Protein Layers»

**Revendications**

**1.** Procédé de mesure de l'effet d'une action sur un film comprenant les étapes suivantes.

a) introduction dans une solution d'au moins une substance capable de former un film,
b) introduction dans la solution obtenue en (a) d'au moins deux particules, lesdites particules reposant sur une surface immergée S dans ladite solution,
c) regroupement des particules sur ladite surface immergée S, lesdites particules formant sur ladite surface un point ou une tache,
d) formation dudit film à partir de ladite substance,
e) observation du point ou de la tache sur la surface S,
f) application d'une action mécanique et/ou physique à ladite solution,
g) observation de l'effet sur le film de l'action appliquée à l'étape (f) par observation du point ou de la tache sur la surface S et,
h) détermination de l'effet de l'action appliquée sur le film par comparaison des observations des étapes (e) et (g) précitées

**2.** Procédé selon la revendication 1 dans lequel l'action est une action choisie parmi une action mécanique ou physique.

**3.** Procédé selon l'une quelconque des revendications précédentes dans lequel la substance capable de former un biofilm est choisie parmi les microorganismes, les aliments, les substances chimiques.

4. Procédé selon l'une quelconque des revendications précédentes dans lequel lesdites, au moins deux, particules sont indépendamment une particule chargée électriquement, magnétique ou magnétisable ou recouverte d'au moins une couche magnétique ou magnétisable.

5. Procédé selon l'une quelconque des revendications précédentes, dans lequel lesdites, au moins deux, particules sont éclairées au moyen d'une source lumineuse pour augmenter le contraste entre la particule et la solution.

**Patentansprüche**

1. Verfahren zum Messen des Effekts eines Einwirkens auf einen Film, umfassend die folgenden Schritte:

   a) Einführung mindestens einer Substanz, die einen Film bilden kann, in eine Lösung,
   b) Einführung mindestens zwei Partikeln in die unter (a) erhaltene Lösung, wobei die Partikeln auf einer in die Lösung eingetauchten Fläche S liegen,
   c) Gruppieren die Partikeln auf der eingetauchten Fläche S, wobei die Partikeln auf der Fläche einen Punkt oder einen Fleck bilden,
   d) Bildung des Films aus der Substanz,
   e) Beobachtung des Punktes oder des Flecks auf der Fläche S,
   f) Ausüben einer mechanischen und/oder physischen Einwirkung auf die Lösung,
   g) Beobachtung des Effekts der in Schritt (f) ausgeübten Einwirkung auf den Film durch Beobachtung des Punktes oder des Flecks auf der Fläche S, und
   h) Bestimmung des Effekts der ausgeübten Einwirkung auf den Film durch Vergleich der Beobachtungen aus den vorgenannten Schritten (e) und (g).

2. Verfahren nach Anspruch 1, bei dem die Einwirkung unter einer mechanischen oder physischen Einwirkung ausgewählt wird.

3. Verfahren nach einem der vorhergehenden Ansprüche, bei dem die Substanz, die geeignet ist, einen Biofilm zu bilden, unter den Mikroorganismen, den Nahrungsmitteln, den chemischen Substanzen ausgewählt ist.

4. Verfahren nach einem der vorhergehenden Ansprüche, bei dem die mindestens zwei Partikeln unabhängig ein elektrisch geladenes, magnetisches oder magnetisierbares oder mit mindestens einer magnetischen oder magnetisierbaren Schicht überzogenes Partikeln sind.

5. Verfahren nach einem der vorhergehenden Ansprüche, bei dem die mindestens zwei Partikeln mit Hilfe einer Leuchtquelle beleuchtet werden, um den Kontrast zwischen dem Partikeln und der Lösung zu erhöhen.

**Claims**

1. A process for measuring the effect of an action on a film comprising the following steps:

   a) introducing, into a solution, at least one substance capable of forming a film,
   b) introducing, into the solution obtained in (a), at least two particles, said particles resting on a submerged surface S in said solution,
   c) regrouping of the particles on said submerged surface S, said particles forming on said surface a spot or a mark,
   d) forming said film from said substance,
   e) observing the spot or mark on the surface S,
   f) applying a mechanical and/or physical action to said solution,
   g) observing the effect on the film of the action applied in step (f) by observing the spot or mark on the surface S, and
   h) determining the effect of the action applied to the film by comparing the observations from the aforementioned steps (e) and (g).

2. The process according to claim 1, wherein the action is an action selected from a mechanical or physical action.

3. The process according to any of the preceding claims, wherein the substance capable of forming a biofilm is chosen from microorganisms, foodstuffs and chemical substances.

4. The process according to any of the preceding claims, wherein said, at least two particles are independently a magnetic, a magnetizable or an electrically charged particle or a particle covered with at least one magnetic or magnetizable layer.

5. The process according to any of the preceding claims, wherein said, at least two, particles are illuminated by means of a light source in order to increase the contrast between the particle and the solution

FIGURE 1A

FIGURE 1 B

FIGURE 2A

FIGURE 2 B

FIGURE 3A

FIGURE 3 B

FIGURE 3 C

FIGURE 4 A

FIGURE 4 B

FIGURE 5 A

FIGURE 5 B

FIGURE 5C

FIGURE 6 A

FIGURE 6 B

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2916761 A1

**Littérature non-brevet citée dans la description**

- **SINGH et al.** Refractive Index Measurement and its Applications. *Physica Scripta,* 2002, vol. 65, 167-180
- **VÔRÔS et al.** The Density and Refractive Index of Adsorbing Protein Layers. *Biophysical Journal,* 2004, vol. 87, 553-561
- *JOURNAL OF MICROBIOLOGICAL METHODS,* 2007, vol. 68 (3), ISBN 0167-7012, 605-612
- Refractive Index Measurement and its Applications. *Physica Scripta.,* 2002, vol. 65, 167-180
- **VÖRÖS et al.** The Density and Refractive Index of Adsorbing Protein Layers. *Biophysical Journal,* 2004, vol. 87, 553-561